# EUROPEAN PATENT SPECIFICATION

(11) **EP 2 103 285 B1**
(45) Date of publication and mention of the grant of the patent: **21.09.2016**
(21) Application number: 09250633.6
(22) Date of filing: 05.03.2009
(51) Int. Cl.: A61F 5/00, A61F 2/02

(54) **System for communicating with an implantable antenna**
System zur Kommunikation mit einer implantierbaren Antenne
Système de communication avec une antenne implantable

(30) Priority: 06.03.2008 US 43616
(43) Date of publication of application: 23.09.2009
(73) Proprietor: Ethicon Endo-Surgery, Inc., Cincinnati, OH 45242 (US)
(72) Inventor: Dlugos, Jr. Daniel F., Middletown, Ohio 45044 (US); Ortiz, Marks S., Milford, Ohio 45150 (US); Marcotte, Amy L., Mason, Ohio 45040 (US); Plescia, David N., Cincinnati, Ohio 45227 (US)
(74) Representative: Tunstall, Christopher Stephen

(56) References cited:
- EP-A- 0 619 101
- EP-A- 1 704 833
- EP-A- 1 992 316
- EP-A2- 2 095 797
- WO-A-2005/105001
- FR-A- 2 869 466
- US-A- 6 009 350

## Description

### FIELD

The present application relates to devices for aligning an antenna implanted under the skin with an external device or an internally delivered device.

### BACKGROUND

Obesity is becoming a growing concern, particularly in the United States, as the number of obese people continues to increase, and more is learned about the negative health effects of obesity. Morbid obesity, in which a person is 100 pounds or more over ideal body weight, in particular poses significant risks for severe health problems. Accordingly, a great deal of attention is being focused on treating obese patients. One method of treating morbid obesity has been to place a restriction device, such as an elongated band, about the upper portion of the stomach. Gastric bands have typically comprised a fluid-filled elastomeric balloon with fixed endpoints that encircles the stomach just inferior to the esophageal-gastric junction to form a small gastric pouch above the band and a reduced stoma opening in the stomach. When fluid is infused into the balloon, the band expands against the stomach creating a food intake restriction or stoma in the stomach. To decrease this restriction, fluid is removed from the band. The effect of the band is to reduce the available stomach volume and thus the amount of food that can be consumed before becoming "full."

Food restriction devices have also comprised mechanically adjusted bands that similarly encircle the upper portion of the stomach. These bands include any number of resilient materials or gearing devices, as well as drive members, for adjusting the bands. Additionally, gastric bands have been developed that include both hydraulic and mechanical drive elements. It is also known to restrict the available food volume in the stomach cavity by implanting an inflatable elastomeric balloon within the stomach cavity itself. The balloon is filled with a fluid to expand against the stomach walls and, thereby, decrease the available food volume within the stomach.

With each of the above-described food restriction devices, safe, effective treatment requires that the device be regularly monitored and adjusted to vary the degree of restriction applied to the stomach. Traditionally, adjusting a gastric band required a scheduled clinician visit during which a Huber needle and syringe were used to penetrate the patient's skin and remove fluid from the balloon via an injection port. More recently, implantable pumps have been developed which enable non-invasive adjustments of the band. An external programmer communicates with the implanted pump using telemetry to control the pump. During a scheduled visit, a physician places a hand-held portion of the programmer near the gastric implant and transmits command signals to the implant. The implant in turn adjusts the band and transmits a response command to the programmer.

Implants such as those described above include electronics, such as an antenna, which are used to transmit information to an external device in order to control adjustment of the band. It is important for the implanted antenna to be properly aligned with the external device to allow for successful information transmissions. It can be difficult and time-consuming to properly align the internal antenna with the external devices as to power the implant and/or transmit data therebetween as the antenna can shift locations and orientations beneath the skin.

Thus. there remains a need for a system capable of aligning an antenna implanted under the skin with an external device or an internally delivered device.

French patent publication no. FR 2869466 relates to a process which involves electrically assembling several transmitting antennae in parallel.

International patent publication no. WO 2005/105001 relates to a gastric ring which comprises at least one receiving antenna, at least one of said antennas being misaligned in relation to the plane of the gastric ring, and an electronic device which is attached to the ring and which is designed to ensure the optimal use of the energy received.

US patent no. 6,009,350 relates to an antenna apparatus for an implantable medical device adapted and disposed to have an increased telemetry range by providing a plurality of antennas connected in parallel with each other, and physically separated from each other.

European patent publication EP 1992316 relates to the problem of accessing an injection port transcutaneously and uses wireless position transducers in an inflation port assembly and in an injection syringe. The measurements provided by the transducers indicate to the practitioner the position and orientation of syringe relative to the injection port.

European patent publication EP 1704833 relates to a restriction system, such as an adjustable gastric band, for forming a restriction in a patient and non-invasively communicating pressure data regarding the restriction to an external monitor. The system includes a restriction device for implantation in a patient to form a restriction. The system further includes an implanted port connected to the restriction device. The port contains a working fluid for affecting the size of the restriction. The system further includes a pressure sensing system in communication with the working fluid for measuring the pressure of the working fluid and transmitting pressure measurement data to an external monitor.

European patent application EP 2095797 forms part of the state of the art under Art 54(3) EPC and discloses a restriction system including a first housing having a reservoir formed therein and configured to receive fluid, and a second housing spaced apart from and in fluid communication with the first housing. The second housing can have a sensor, for example, for measuring fluid pressure. A restriction device can be in fluid communication with the first and second housings and can be adapted to form a restriction in a pathway.

### SUMMARY

The present invention relates to an implantable restriction system as defined in independent claim 1. Preferred embodiments are defined in the dependent claims. In particular, an implantable restriction system is provided and includes an implantable restriction device configured to form a restriction in a pathway, and an implantable housing associated with the implantable restriction device. The housing has at least one antenna that is configured to communicate telemetrically with a transceiver regardless of a rotational orientation of the housing about a longitudinal axis of the catheter. The at least one antenna extends along an axis aligned with the longitudinal axis of a catheter extending from the housing. The transceiver can have a variety of forms. For example, the transceiver can be an external device located adjacent to a tissue surface, or the transceiver can be disposed on a device that can be configured to be delivered internally within a patient's body. In one embodiment, the implantable housing can contain a sensor that can be configured, for example, to measure at least one of a system parameter and a physiological parameter, and the antenna can be effective to communicate the measured parameter to the transceiver. The at least one antenna can also be configured to receive energy to power the sensor, or data, or other information.

The antenna can be positioned in the housing in a variety of ways. For example, the implantable housing can include a support disposed therein having proximal and distal ends and extending along the longitudinal axis of the catheter. In an exemplary embodiment, the at least one antenna can include a plurality of antennae with each antenna disposed around the proximal and distal ends of the support and spaced radially about the support from an adjacent antenna. The antenna can be spaced around the support in a number of configurations. For example, each of the plurality of antennae can be spaced radially apart from one another, such as by about 180 degrees, about 120 degrees, about 90 degrees, or about 60 degrees, or at some other angular increment. In another exemplary embodiment, the at least one antenna can be in the form of a cylindrical coil antenna.

In another embodiment, a restriction system is provided and includes an implantable band configured to form a restriction in a pathway, and a housing associated with the band and having a catheter extending therefrom defining a longitudinal axis along a length thereof. An implantable sensor can be configured to measure at least one of a restriction system parameter and a physiological parameter, for example a fluid pressure of fluid in the band. At least one antenna can be associated with the housing and configured to emit a magnetic field toward an external device positioned on a tissue surface directly adjacent the housing regardless of a rotational orientation of the housing about an axis of the catheter extending from the housing. The antenna can have a variety of configurations, including a plurality of antennae extending along an axis aligned with the longitudinal axis of the catheter, and a cylindrical coil antenna having a longitudinal axis that is aligned with the longitudinal axis of the catheter.

### BRIEF DESCRIPTION OF THE DRAWINGS

The invention will be more fully understood from the following detailed description taken in conjunction with the accompanying drawings, in which:
FIG. 1A is a schematic diagram of an embodiment of a food intake restriction system;
FIG. 1B is perspective view of an embodiment of an implantable portion of the food intake restriction system of FIG. 1A;
FIG. 2A is a perspective view of the food intake restriction device of FIG. 1A;
FIG. 2B is a schematic diagram of the food intake restriction device of FIG. 2A applied about the gastro-esophageal junction of a patient;
FIG. 3 is a perspective view of an embodiment of the injection port housing of FIG. 1A;
FIG. 4 is a perspective view of an embodiment of the sensor housing of FIG. 1A;
FIG. 5 illustrates an embodiment of the sensor housing of FIG. 1A;
FIG. 6 is a schematic of an embodiment of a variable resistance circuit for the pressure sensor of FIG. 5;
FIG. 7 is a block diagram showing an embodiment of internal and external components of the food intake restriction device of FIG. 1A;
FIG. 8 is a perspective view of one embodiment of the restriction system of FIG. 1A-1B showing a sensor housing including a plurality of antenna disposed therein;
FIG. 9 is a perspective view of one embodiment of a support for supporting the antenna disposed in the housing of FIG. 8;
FIG. 10 is a perspective view of another embodiment of a support for supporting the antenna disposed in the housing of FIG. 8;
FIG. 11 is a perspective view of another embodiment of an antenna configured to be disposed in a sensor housing;
FIG. 12 is a perspective view of a sensor housing including the antenna of FIG. 11;
FIG. 13 is a perspective view of another embodiment of the restriction system of FIG. 1A-1B showing a housing including a plurality of antenna disposed therein; and
FIG. 14 is a perspective view of the embodiment of the housing of FIG. 13 showing another embodiment of an antenna disposed therein.

### DETAILED DESCRIPTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Various exemplary devices are provided for communicating with an implantable restriction system. In one embodiment, the implantable restriction system includes a
housing having at least one internal antenna that can be in communication with an implantable sensor configured to measure system parameters (e.g., pressure) and/or physiological parameters. The internal antenna can be configured to emit a magnetic field toward an external device or an internally delivered device regardless of the rotational orientation of the housing about a longitudinal axis of a catheter extending from the housing to allow communication with the external device or the internally delivered device, for example, to transmit power to the implantable sensor and/or transfer and/or receive data between the internal antenna and the external or internally delivered device.

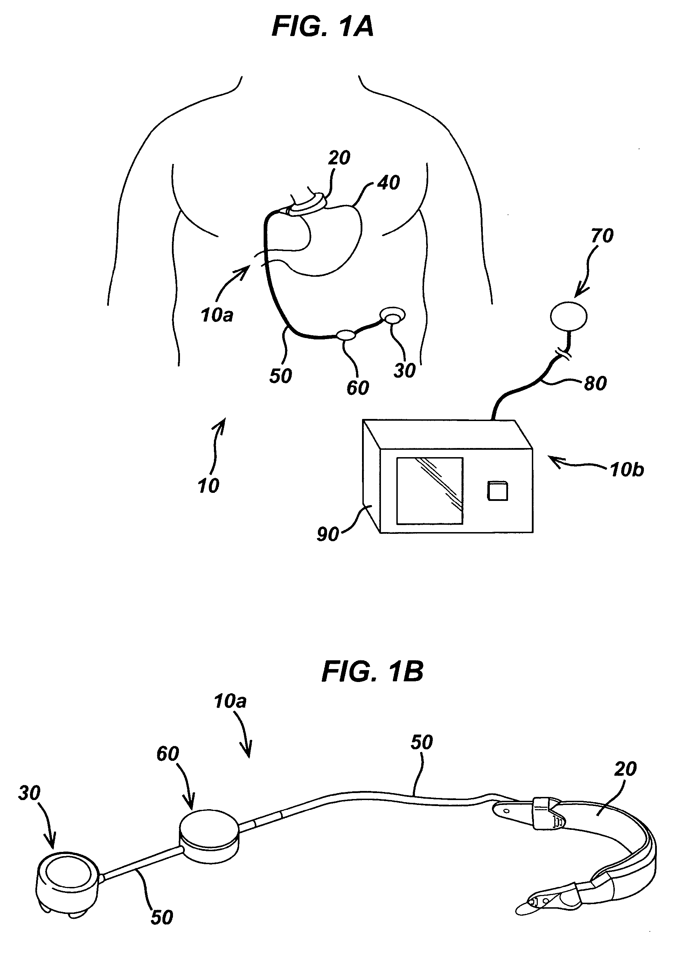

While the present invention can be used with a variety of restriction systems known in the art, FIG. 1A illustrates one exemplary embodiment of a food intake restriction system 10 in use in a patient. As shown, the system 10 generally includes an implantable portion 10a and an external portion 10b. FIG. 1B illustrates the implantable portion 10a outside of a patient. As shown, the implantable portion 10a includes an adjustable gastric band 20 that is configured to be positioned around the upper portion of a patient's stomach 40 and an injection port housing 30 that is fluidly coupled to the adjustable gastric band 20, e.g., via a catheter 50. The injection port 30 is configured to allow fluid to be introduced into and removed from the gastric band 20 to thereby adjust the size of the band 20 and thus the pressure applied to the stomach 40. The injection port 30 can thus be implanted at a location within the body that is accessible through tissue. Typically, injection ports are positioned in the lateral subcostal region of the patient's abdomen under the skin and layers of fatty tissue. Surgeons also typically implant injection ports on the sternum of the patient.

The internal portion 10a can also include a sensing or measuring device that is in fluid communication with the closed fluid circuit in the implantable portion 10a. In one embodiment, the sensing device is a pressure sensing device configured to measure the fluid pressure of the closed fluid circuit. While the pressure measuring device can have various configurations and can be positioned anywhere along the internal portion 10a, including within the injection port 30 and as described further below, in the illustrated embodiment the pressure measuring device is in the form of a pressure sensor that is disposed within a sensor housing 60 positioned adjacent to the injection port 30. The catheter 50 can include a first portion that is coupled between the gastric band 20 and the pressure sensor housing 60 and a second portion that is coupled between the pressure sensor housing 60 and the injection port 30. While it is understood that the sensing device can be configured to obtain data relating to one or more relevant parameters, including
physiological parameters, generally it will be described herein in a context of a pressure sensing device.

As further shown in FIG. 1A, the external portion 10b generally includes a data reading device 70 that is configured to be positioned on the skin surface above the pressure sensor housing 60 (which can be implanted beneath thick tissue, e.g., over 10 cm thick) to non-invasively communicate (as described in detail below) with the pressure sensor housing 60 and thereby obtain pressure measurements. The data reading device 70 can optionally be electrically coupled (wirelessly or wired, as in this embodiment via an electrical cable assembly 80) to a control box 90 that can display the pressure measurements, other data obtained from the data reading device 70, and/or data alerts. While shown in this example as being local to the patient, the control box 90 can be at a location local to or remote from the patient.

In some embodiments, the external portion 10b can include a sensing system configured to obtain data related to one or more relevant parameters, such as fluid pressure of the closed fluid circuit of the internal portion 10a. For example, pressure in the closed fluid circuit can be measured through a Huber needle in fluid communication with the injection port 30. An exemplary external pressure reading system is described in U.S. Publication No. 2006/0211912, entitled "External Pressure-Based Gastric Band Adjustment System and Method".

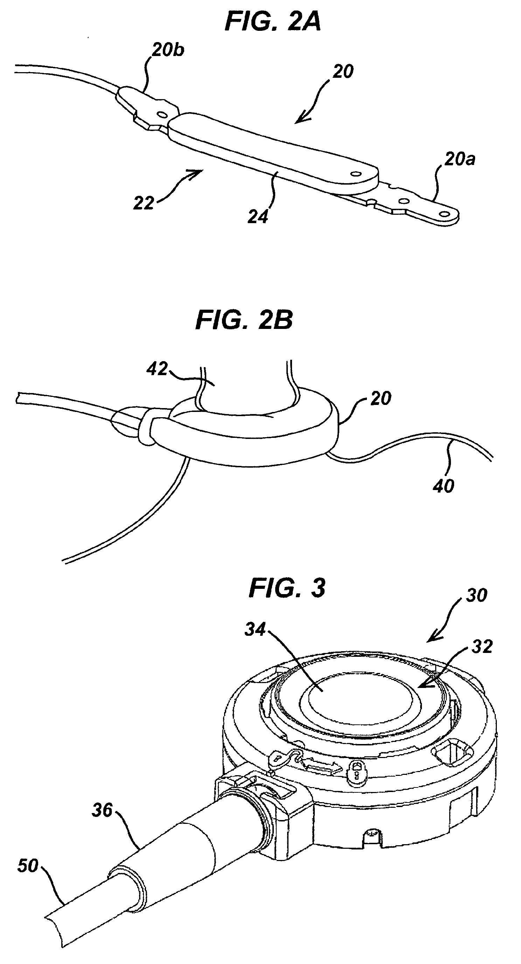

FIG. 2A shows the gastric band 20 in more detail. While the gastric band 20 can have a variety of configurations, and various gastric bands currently known in the art can be used with the present disclosure, in the illustrated embodiment the gastric band 20 has a generally elongate shape with a support structure 22 having first and second opposite ends 20a, 20b that can be formed in a loop such that the ends are secured to each other. Various mating techniques can be used to secure the ends 20a, 20b to one another. In the illustrated embodiment, the ends 20a, 20b are in the form of straps that mate together, with one laying on top of the other. In another embodiment, illustrated, for example, in FIGS. 1B and 2B, a support structure at one end of the gastric band 20 can include an opening through which the other end of the gastric band 20 can feed through to secure the ends to one another. The gastric band 20 can also include a variable volume member, such as an inflatable balloon 24, that is disposed or formed on one side of the support structure 22 and that is configured to be positioned adjacent to tissue. The balloon 24
can expand or contract against the outer wall of the stomach to form an adjustable stoma for controllably restricting food intake into the stomach.

A person skilled in the art will appreciate that the gastric band can have a variety of other configurations. Moreover, the various methods and devices disclosed herein have equal applicability to other types of implantable bands. For example, bands are used for the treatment of fecal incontinence, as described in U.S. Pat. No. 6,461,292. Bands can also be used to treat urinary incontinence, as described in U.S. Publication No. 2003/0105385. Bands can also be used to treat heartburn and/or acid reflux, as disclosed in U.S. Pat. No. 6,470,892. Bands can also be used to treat impotence, as described in U.S. Publication No. 2003/0114729.

FIG. 2B shows the adjustable gastric band 20 applied about the gastro-esophageal junction of a patient. As shown, the band 20 at least substantially encloses the upper portion of the stomach 40 near the junction with the patient's esophagus 42. After the band 20 is implanted, preferably in the deflated configuration wherein the band 20 contains little or no fluid, the band 20 can be inflated, e.g., using saline, to decrease the size of the stoma opening. A person skilled in the art will appreciate that various techniques, including mechanical and electrical techniques, can be used to adjust the band 20. FIG. 2B also shows an alternate location of a sensing device 41, disposed in a buckle 43 of the band 20.

The fluid injection port 30 can also have a variety of configurations. In the embodiment shown in FIG. 3, the injection port 30 has a generally cylindrical housing with a distal or bottom surface and a perimeter wall extending proximally from the bottom surface and defining a proximal opening 32. The proximal opening 32 can include a needle-penetrable septum 34 extending there across and providing access to a fluid reservoir (not visible in FIG. 3) formed within the housing. The septum 34 is preferably placed in a proximal enough position such that the depth of the reservoir is sufficient enough to expose the open tip of a needle, such as a Huber needle, so that fluid transfer can take place. The septum 34 is preferably arranged so that it will self seal after being punctured by a needle and the needle is withdrawn. As further shown in FIG. 3, the port 30 can further include a catheter tube connection member 36 that is in fluid communication with the reservoir and that is configured to couple to a catheter (e.g., the catheter
50). A person skilled in the art will appreciate that the housing can be made from any number of materials, including stainless steel, titanium, ceramic, glass, and polymeric materials, and the septum 34 can likewise be made from any number of materials, including silicone.

The reading device 70 can also have a variety of configurations, and one exemplary pressure reading device is disclosed in more detail in commonly-owned U.S. Publication No. 2006/0189888 and U.S. Publication No. 2006/0199997. In general, the reading device 70 can non-invasively measure the pressure of the fluid within the implanted portion 10a even when the pressure sensing device is implanted beneath thick (at least over 10 cm, and possibly over 15 cm) subcutaneous fat tissue. The physician can hold the reading device 70 against the patient's skin near the location of the sensor housing 60 and/or other pressure sensing device location(s), obtain sensed pressure data and possibly other information as discussed herein, and observe the pressure reading (and/or other data) on a display on the control box 90. The data reading device 70 can also be removably attached to the patient, as discussed further below, such as during a prolonged examination, using straps, adhesives, and other well-known methods. The data reading device 70 can operate through conventional cloth or paper surgical drapes, and can also include a disposal cover (not shown) that may be replaced for each patient.

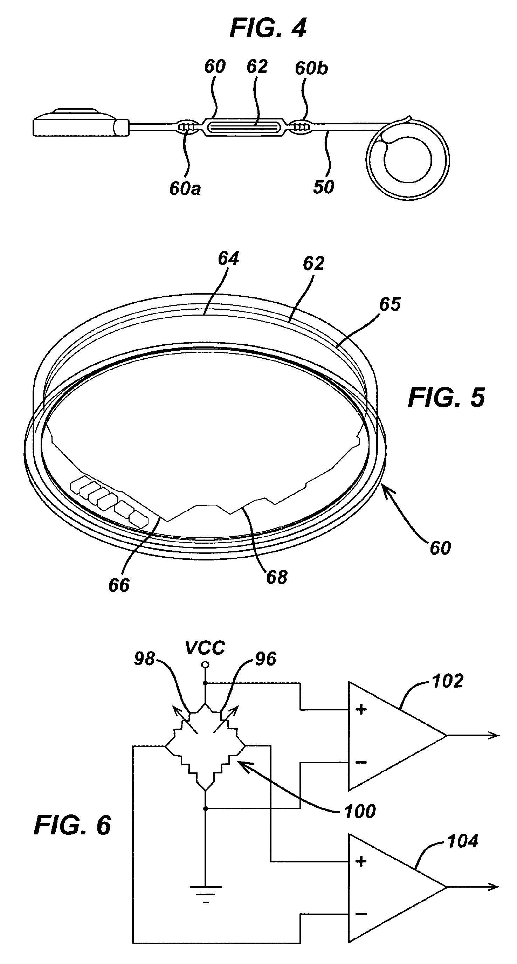

As indicated above, the system 10 can also include one or more sensors for monitoring the operation of the gastric restriction system 10. The sensor(s) can be configured to measure various operational parameters of the system 10 including, but not limited to, a pressure within the system, a temperature within the system, a peristaltic pulse event or frequency, the peristaltic pulse width, the peristaltic pulse duration, and the peristaltic pulse amplitude. In one exemplary embodiment, the system can include a sensor in the form of a pressure measuring device that is in communication with the closed fluid circuit and that is configured to measure the fluid pressure within the system, which corresponds to the amount of restriction applied by the adjustable gastric band to the patient's stomach. The sensor can also be configured to measure a variety of other parameters, for example, pulse count and pulse width. In use, measuring the fluid pressure, or any other control parameter of the system, can enable a physician (or other medical professionals) to evaluate the performance of the restriction system. In the illustrated embodiment, shown in FIG. 4, the pressure measuring device is in the form of a pressure sensor 62 disposed within the sensor housing 60. The pressure measuring device can, however, be
disposed anywhere within the closed hydraulic circuit of the implantable portion, and various exemplary locations and configurations are disclosed in more detail in commonly-owned U.S. Publication No. 2006/0211913 entitled "Non-Invasive Pressure Measurement In a Fluid Adjustable Restrictive Device," filed on March 7, 2006. In general, the illustrated sensor housing 60 includes an inlet 60a and an outlet 60b that are in fluid communication with the fluid in the implantable portion 10a. An already-implanted catheter 50 can be retrofitted with the sensor housing 60, such as by severing the catheter 50 and inserting barbed connectors (or any other connectors, such as clamps, clips, adhesives, welding, etc.) into the severed ends of the catheter 50. The sensor 62 can be disposed within the housing 60 and be configured to respond to fluid pressure changes within the hydraulic circuit and convert the pressure changes into a usable form of data.

Various pressure sensors known in the art can be used as the pressure sensor 62, such as a wireless pressure sensor provided by CardioMEMS, Inc. of Atlanta, Georgia, though a suitable Micro-Electro-Mechanical Systems ("MEMS") pressure sensor may be obtained from any other source, including but not limited to Integrated Sensing Systems, Inc. (ISSYS) of Ypsilanti, Michigan and Remon Medical Technologies, Inc. of Waltham, Massachusetts. One exemplary MEMS pressure sensor is described in U.S. Patent No. 6,855,115. It will also be appreciated by a person skilled in the art that suitable pressure sensors can include, but are not limited to, capacitive, piezoresistive, silicon strain gauge, or ultrasonic (acoustic) pressure sensors, as well as various other devices capable of measuring pressure.

One embodiment of a configuration of the sensor housing 60 having the sensor 62 disposed within it is shown in FIG. 5. The sensor housing 60 in this example can be made of a two piece construction including a circuit board, which can be made of a hermetic material to serve as a hermetic component (bottom), and a hermetic top of compatible material bonded together to prevent fluid from contacting any elements disposed within the sensor housing 60, except as discussed for the sensor 62. The sensor housing 60 can be made from any biocompatible material appropriate for use in a body, such as a polymer, biocompatible metal, ceramic, glass, and other similar types of material. Furthermore, the sensor housing 60 can be made from any one or more of transparent (as shown in FIG. 5), opaque, semi-opaque, and radio-opaque materials. A circuit board 64 including, among other elements, a microcontroller
65 (e.g., a processor), can also be disposed within the housing 60 to help process and communicate pressure measurements gathered by the sensor 62, and also possibly other data related to the band 20. (The circuit board 64 can also be part of the housing 60, as mentioned above.) As further discussed below, the circuit board 64 also includes a transcutaneous energy transfer (TET)/telemetry coil and a capacitor. Optionally, a temperature sensor can be integrated into the circuit board 64. The microcontroller 65, the TET/telemetry coil, the capacitor, and/or the temperature sensor can be in communication via the circuit board 64 or via any other suitable component(s). The TET/telemetry coil and capacitor can collectively form a tuned tank circuit for receiving power from the external portion 10b and transmitting pressure measurements to a pressure reading device, e.g., the reading device 70. Moreover, to the extent that a telemetry component associated with the pressure sensor 62 is unable to reach a telemetry device external to the patient without some assistance, such assistance can be provided by any suitable number of relays (not shown) or other devices.

In use, fluid can enter the sensor housing 60 through an opening 66 located anywhere on the housing's surface (here, the bottom surface) and come into contact with a pressure sensing surface 68 of the sensor 62. The sensor 62 is typically hermetically sealed to the motherboard such that fluid entering the opening 66 cannot infiltrate and affect operation of the sensor 62 except at the pressure sensing surface 68. The sensor 62 can measure the pressure of fluid coming into contact with the pressure sensing surface 68 as fluid flows in and out of the opening 66. For example, the pressure sensing surface 68 can include a diaphragm having a deformable surface such that when fluid flows through the opening 66, the fluid impacts the surface of the diaphragm, causing the surface to mechanically displace. The mechanical displacement of the diaphragm can be converted to an electrical signal by a variable resistance circuit including a pair of variable resistance, silicon strain gauges. One strain gauge can be attached to a center portion of diaphragm to measure the displacement of the diaphragm, while the second, matched strain gauge can be attached near the outer edge of diaphragm. The strain gauges can be attached to the diaphragm with adhesives or can be diffused into the diaphragm structure. As fluid pressure within band 20 fluctuates, the surface of the diaphragm can deform up or down, thereby producing a resistance change in the center strain gauge.

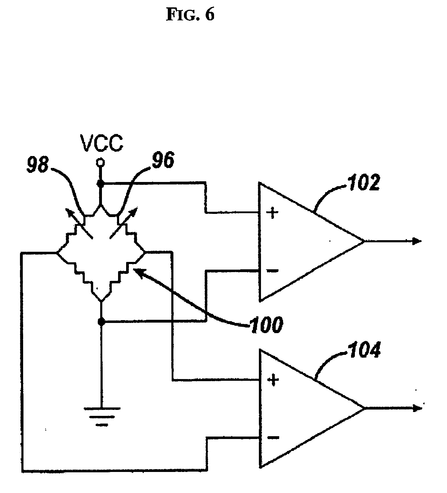

One embodiment of a variable resistance circuit for the sensor 62 is shown in FIG. 6. The circuit includes first and second strain gauges 96, 98 that form the top two resistance elements of a half-compensated, Wheatstone bridge circuit 100. As the first strain gauge 96 reacts to the mechanical displacements of the sensor's diaphragm, the changing resistance of the first gauge 96 changes the potential across the top portion of the bridge circuit 100. The second strain gauge 98 is matched to the first strain gauge 96 and athermalizes the Wheatstone bridge circuit 100. First and second differential amplifiers 102, 104 are connected to the bridge circuit 100 to measure the change in potential within the bridge circuit 100 due to the variable resistance strain gauges 96, 98. In particular, the first differential amplifier 102 measures the voltage across the entire bridge circuit 100, while the second differential amplifier 104 measures the differential voltage across the strain gauge half of bridge circuit 100. The greater the differential between the strain gauge voltages, for a fixed voltage across the bridge, the greater the pressure difference. Output signals from the differential amplifiers 102, 104 can be applied to the microcontroller 65 integrated into the circuit board 64, and the microcontroller 65 can transmit the measured pressure data to a device external to the patient. If desired, a fully compensated Wheatstone bridge circuit can also be used to increase the sensitivity and accuracy of the pressure sensor 62. In a fully compensated bridge circuit, four strain gauges are attached to the surface of diaphragm rather than only two strain gauges.

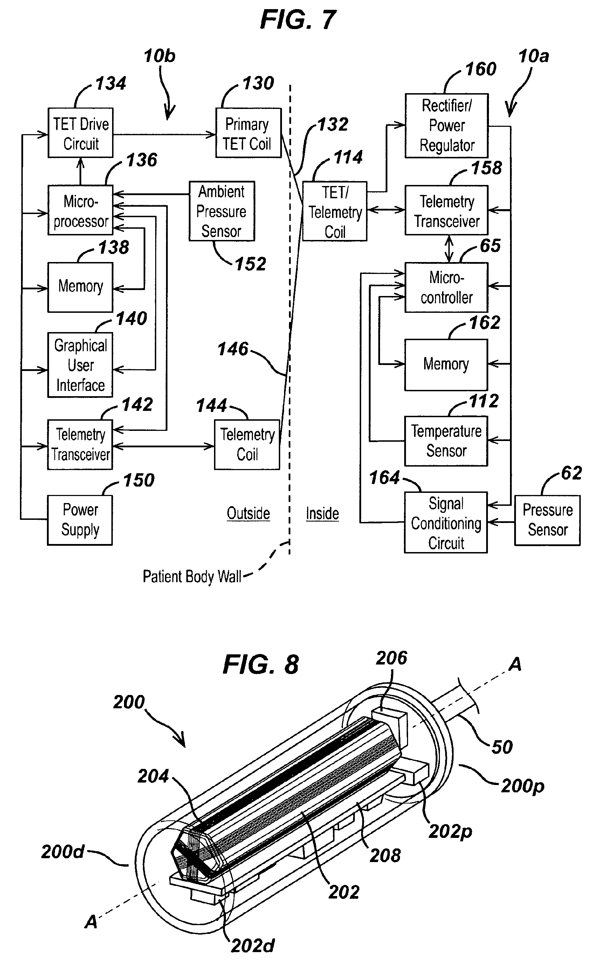

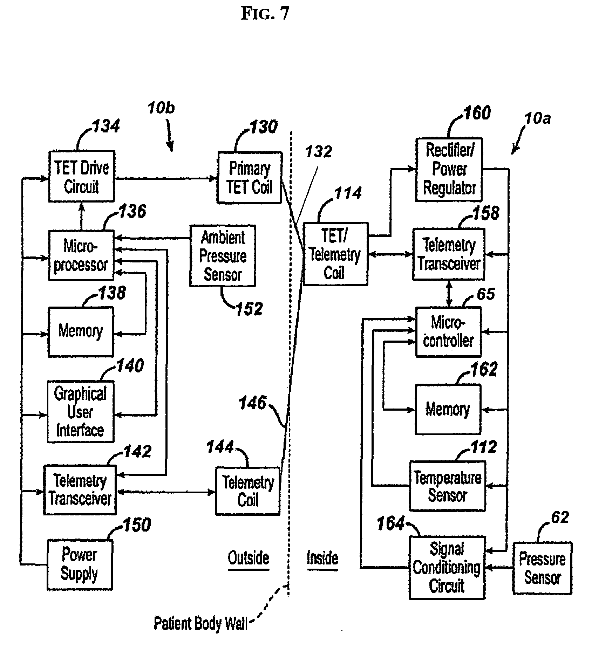

FIG. 7 illustrates one embodiment of components included in the internal and external portions 10a, 10b. As shown in FIG. 7, the external portion 10b includes a primary TET coil 130 for transmitting a power signal 132 to the internal portion 10a. A telemetry coil 144 is also included for transmitting data signals to the internal portion 10a. The primary TET coil 130 and the telemetry coil 144 combine to form an antenna, e.g., the reading device 70. The external portion 10b, e.g., disposed in the control box 90, includes a TET drive circuit 134 for controlling the application of power to the primary TET coil 130. The TET drive circuit 134 is controlled by a microprocessor 136 having an associated memory 138. A graphical user interface 140 is connected to the microprocessor 136 for inputting patient information, displaying data and physician instructions, and/or printing data and physician instructions. Through the use of interface 140, a user such as the patient or a clinician can transmit an adjustment request to the physician and also enter reasons for the request. Additionally, the user interface 140 can enable the patient to read and respond to instructions from the physician and/or pressure measurement alerts, as discussed further below.

The external portion 10b also includes a primary telemetry transceiver 142 for transmitting interrogation commands to and receiving response data, including sensed pressure data, from the implanted microcontroller 65. The primary transceiver 142 is electrically connected to the microprocessor 136 for inputting and receiving command and data signals. The primary transceiver 142 drives the telemetry coil 144 to resonate at a selected RF communication frequency. The resonating circuit can generate a downlink alternating magnetic field 146 that transmits command data to the microcontroller 65. Alternatively, the transceiver 142 can receive telemetry signals transmitted from a secondary TET/telemetry coil 114 in the internal portion 10a. The received data can be stored in the memory 138 associated with the microprocessor 136. A power supply 150 can supply energy to the control box 90 in order to power element(s) in the internal portion 10a. An ambient pressure sensor 152 is connected to microprocessor 136. The microprocessor 136 can use a signal from the ambient pressure sensor 152 to adjust the received pressure measurements for variations in atmospheric pressure due to, for example, variations in barometric conditions or altitude, in order to increase the accuracy of pressure measurements.

FIG. 7 also illustrates components of the internal portion 10a, which in this embodiment are included in the sensor housing 60 (e.g., on the circuit board 64). As shown in FIG. 7, the secondary TET/telemetry coil 114 receives the power/communication signal 132 from the external antenna. The secondary coil 114 forms a tuned tank circuit that is inductively coupled with either the primary TET coil 130 to power the implant or the primary telemetry coil 144 to receive and transmit data. A telemetry transceiver 158 controls data exchange with the secondary coil 114. Additionally, the internal portion 10a includes a rectifier/power regulator 160, the microcontroller 65, a memory 162 associated with the microcontroller 65, a temperature sensor 112, the pressure sensor 62, and a signal conditioning circuit 164. The implanted components can transmit pressure measurements (with or without adjustments due to temperature, etc.) from the sensor 62 to the control box 90 via the antenna (the primary TET coil 130 and the telemetry coil 144). Pressure measurements can be stored in the memory 138, adjusted for ambient pressure, shown on a display on the control box 90, and/or transmitted, possibly in real time, to a remote monitoring station at a location remote from the patient.

As indicated above, the sensor housing can include at least at one antenna that can be configured to allow the implantable restriction system 10 to be powered by and/or communicate with an external device or an internally delivered device. The antenna is disposed in the housing in such a way as to allow effective communication between the antenna and an external device located adjacent to a skin surface or a device configured to be delivered internally within a patient's body, for example, to the gastro-intestinal tract. In particular, the antenna is disposed in a housing to allow the antenna to emit a magnetic field towards the external device or the internally delivered device regardless of the rotational orientation of the housing about a longitudinal axis of the catheter. This can be achieved by orienting the antenna parallel to a longitudinal axis of a catheter extending from the housing.

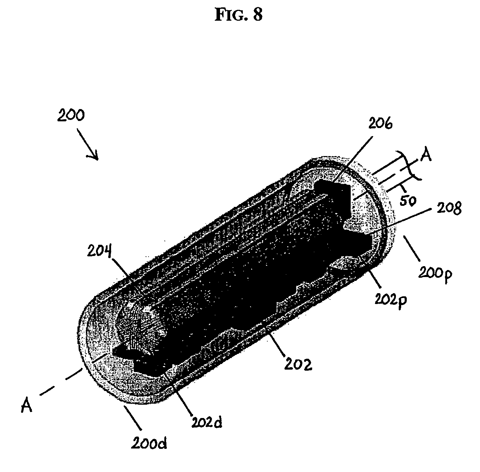

While the housing that contains the antenna, such as sensor housing 60 described above, is shown in FIG. 1B to have a disc-like configuration and in FIG. 4 to have an elongate configuration, the housing can have a variety of configurations, including circular and rectangular configurations. In an exemplary embodiment, shown in FIG. 8, a housing 200 can have a generally elongate cylindrical configuration having proximal and distal ends 200p, 200d that define a longitudinal axis therebetween. A person skilled in the art will appreciate that the housing 200 can have any shape and size but it is preferably configured to be implanted in tissue and to contain at least one antenna 204 disposed therein. The housing 200 can also include a catheter, such as catheter 50, extending therefrom. The catheter 50 can be coupled to the housing using an inlet and/or an outlet that are in fluid communication with the fluid in the implantable portion 10a. In order to allow for effective communication between the antenna 204 and an external device, the antenna 204 extends within the housing 200 along an axis A aligned with a longitudinal axis of the catheter 50. A person skilled in the art will appreciate that aligning the antenna 204 with the longitudinal axis of the catheter 50 includes the antenna 204 being co-axial with or parallel to the longitudinal axis of the catheter 50. Thus, regardless of the rotational orientation of the housing 200 about the longitudinal axis of the catheter 50, the antenna 204 can emit a magnetic field towards a predefined location on a tissue surface to allow the antenna 204 to communicate with the external device. A person skilled in the art will appreciate that the housing 200 can have any configuration so long as the antenna 204 can be positioned therein. Moreover, a person skilled in the art will appreciate that although the housing and the catheter are shown as being arranged in line, in accordance with the present disclosure the components can be arranged in a variety of other ways not in accordance with the present invention, including in a T-configuration or a Y-configuration, and various exemplary configurations are disclosed in more detail in commonly-owned U.S. Publication No. 2006/0211913 entitled "Non-Invasive Pressure Measurement In a Fluid Adjustable Restrictive Device," filed on March 7, 2006.

The housing 200 can also include circuitry, as described above in FIG. 5, that can be disposed in the housing in a variety of ways. For example, in an exemplary embodiment, the circuitry can be anchored in the housing 200 using an attachment member 208 that is configured to couple the circuitry to the proximal end 200p of the housing 200. A person skilled in the art will appreciate, however, that the circuitry can be disposed in the housing 200 in any manner and can be anchored to the housing 200 using any known means.

The at least one antenna 204 can also be disposed within the housing 200 in the housing in a variety of ways. In one embodiment, the housing 200 can include a support 202 disposed therein and configured to support the antenna 204. The support 202 can have a variety of configurations, and can include proximal and distal ends 202p, 202d that define a longitudinal axis therebetween that is parallel to or co-axial with the longitudinal axis of the catheter 50 extending from the housing 200. In the illustrated embodiment, the proximal end 202p of the support 202 is coupled to the proximal end 200p of the housing 200 using an attachment member 206 that is configured to couple the support 202 to an inner proximal wall of the housing 200. A person skilled in the art will appreciate, however, that the support 202 can be coupled to the housing 200 using a variety of techniques. For example, the support 202 can be fixedly coupled to the housing 200 using, for example, adhesives or fasteners, or the support 202 can be removably coupled to the housing 200. A person skilled in the art will appreciate that the support 202 can be coupled to the housing 200 in any way that allows the antenna 204 to be positioned along the support 202. The support 202 can also include features to accommodate any number of antennae 204 configured in any manner along the support 202, as will be discussed in more detail below.

In order to facilitate communication with a device, such as a transceiver, that can be an external device or a device configured to be delivered internally within the body, such as in the gastro-intestinal tract, the housing 200 can include any number of antennae 204 in a variety of configurations to emit and/or receive field lines that are directed towards a tissue surface regardless of the orientation of the housing 200 about the axis of the
catheter 50 extending from the housing 200. In one exemplary embodiment, this allows the antenna 204 to communicate with any device, including external and internal devices, regardless of the orientation of the housing 200 about an axis of the catheter 50 extending from the housing as the housing 200 rotates and/or flips about the axis of the catheter 50 when it is implanted. A person skilled in the art will appreciate that in accordance with the present invention the housing 200 can include a plurality of antennae positioned in any configuration as long each antenna 204 is oriented substantially parallel to the longitudinal axis of the catheter 50 to allow the antennae 204 to emit magnetic field lines towards a location on a tissue surface about the housing 200 to facilitate communication with an external device or an internally delivered device.

For example, in one exemplary embodiment, the housing 200 can include a plurality of antennae 204 disposed around the proximal and distal ends 202p, 202d of the support 202 and spaced radially therearound in order to emit fields lines that allow the antennae 204 to communicate with the external device. The plurality of antennae can be spaced radially apart from one another by any angular increment, such as about 180 degrees, 120 degrees, 90 degrees, 60 degrees, 30 degrees, or some other increment. In the exemplary embodiment of FIG. 8, the first, second, and third antennae can be looped about the support 202 extending along the longitudinal axis thereof and are spaced radially apart from one another by about 120 degrees. In other words, each of the first, second, and third antenna can have a first portion extending along a side of the support 202 and a second portion extending along an opposed side of the support 202. Thus, each of the portions of the first, second, and third antenna are spaced 60 degrees apart from one another.

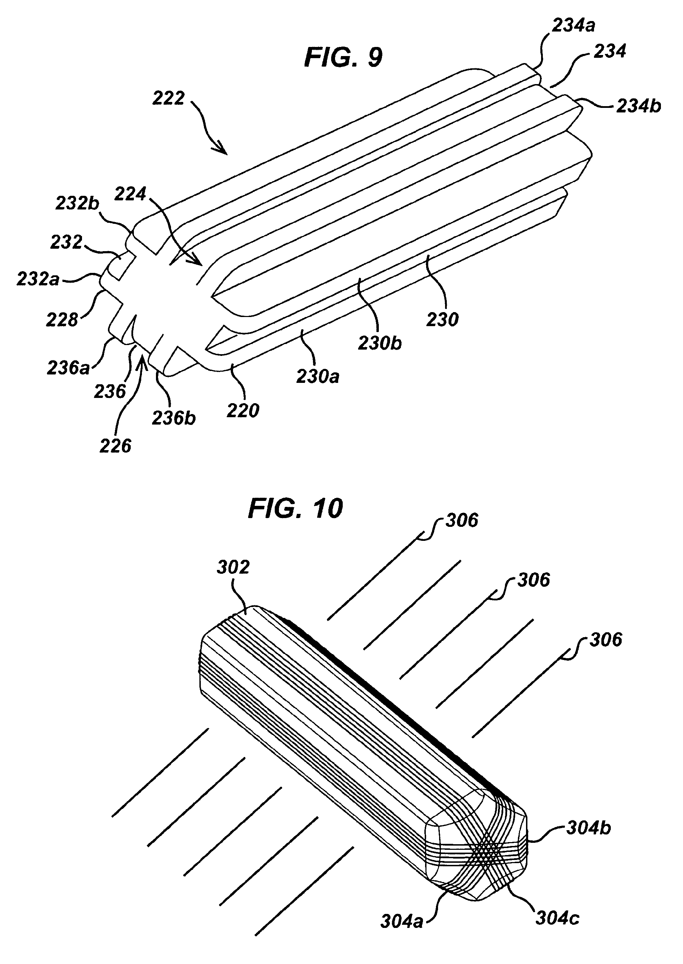

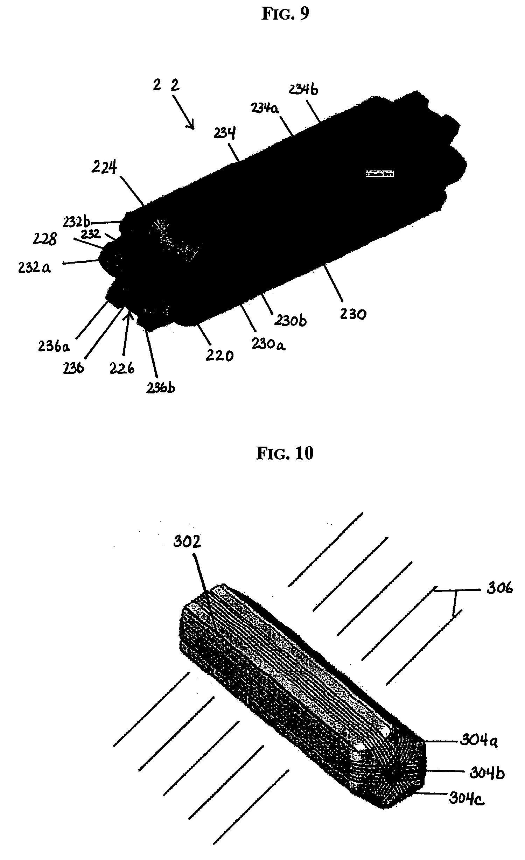

The support can be also have a variety of configurations to support a plurality of antennae spaced radially therearound. For example, FIG. 9 illustrates one exemplary embodiment of a support 222 adapted to support first and second antennae. The first and second antennae can be looped about the support 222 extending along the longitudinal axis thereof and can be spaced radially apart from one another by about 180 degrees. In other words, each of the first and second antenna can have a first portion extending along the a side 220, 224 of the support 222 and a second portion extending along an opposed side 226, 228 of the support 222. Thus, each of the portions of the first and second antenna are spaced 90 degrees apart from one another. In order to accommodate the first and second antenna, the support 222 can have a generally elongate cross-shaped configuration. The support 222 can also include first and second opposed
mounting grooves 230, 232 that are positioned opposite from each other along the sides 220, 228 of the support 222 to support the first antenna, and third and fourth mounting grooves 234, 236 that are positioned opposite from each other and at 90 degrees from the first and second opposed mounting grooves 230, 232 along the sides 224, 226 of the support 222 to support the second antenna. The mounting grooves 230, 232, 234, 236 can have a variety of configurations, but in the illustrated embodiment, are in the form of channels formed along the length of the sides 220, 224, 226, 228 of the support 222 and that are sized and shaped to receive the first and second antennae therein. Each mounting groove 230, 232, 234, 236 can include first and second opposed sidewalls 230a, 230b, 232a, 232b, 234a, 234b, 236a, 236b, and the sidewalls can have a height that prevents the antenna from sliding out of the mounting grooves 230, 232, 234, 236 to hold the first and second antennae in place therein. A person skilled in the art will appreciate that the support 222 can have a variety of configurations to support the first and second antenna. For example, the support 222 can be in form of an elongate rectangle (not shown) having four sides with the mounting grooves 230, 232, 234, 236 formed in each of the sides of the elongate rectangle. Moreover, a person skilled in the art will appreciate that the support 222 can support the antenna without the use of the mounting grooves.

In another exemplary embodiment, first, second, and third antennae 304a, 304b, 304c can be spaced radially apart from one another by about 120 degrees. As shown in FIG. 10, a support 302 can be configured to accommodate the first, second, and third antenna and can have a generally hexagonical shape having six sides. Each pair of opposed sides of the support 302 can hold one of the first, second, and third antenna 304a, 304b, 304c along its length such that the antenna segments are spaced apart from one another at about 60 degrees increments. A person skilled in the art will appreciate that the support 302 can have variety of configurations and include a variety of additional features to support the first, second and third antenna 304a, 304b, 304c. For example, the support 302 can include mounting grooves as describe above with respect to FIG. 9 formed along each of the six sides of the support 302 to hold the first, second, and third antenna 304a, 304b, 304c therein and prevent the first, second, and third antenna 304a, 304b, 304c from sliding on the sides of the support 302. Field lines 306 created by the first, second, and third antenna 304a, 304b, 304c run perpendicular to the longitudinal axis of the support 302 and the housing in which the antenna 304a, 304b, 304c and support 302 are disposed. Thus, when an external device is positioned adjacent to a skin surface or an internal device configured to be delivered internally within the body in order to communicate with the antennae 304a, 304b, 304c, an antenna or other receiver/transmitter of the external device will align with the field lines 306 regardless of the orientation of the antenna 304a, 304b, 304c beneath the skin to allow for communication between the antenna 304a, 304b, 304c and the external or internal device.

A person skilled in the art will appreciate that the antenna can have any configuration and can be configured to emit a field in all directions. For example, the antenna illustrated in FIGS. 8-10 are all configured to emit a field in all directions due to the looping of the antenna around the ends of the support. While the field emitted from the ends of the antenna can be weaker than the field emitted from the portions of the antenna extending along the length of the support, these antenna configurations will emit a field in all directions. In another exemplary embodiment, in order to achieve an antenna that emits a substantially equal field in all directions, the antenna can be formed to have a symmetrical configuration, for example, in the shape of a cube. This allows the antenna to emit a field of substantially the same magnitude in all directions regardless of the rotational orientation of the antenna about a longitudinal axis of the catheter.

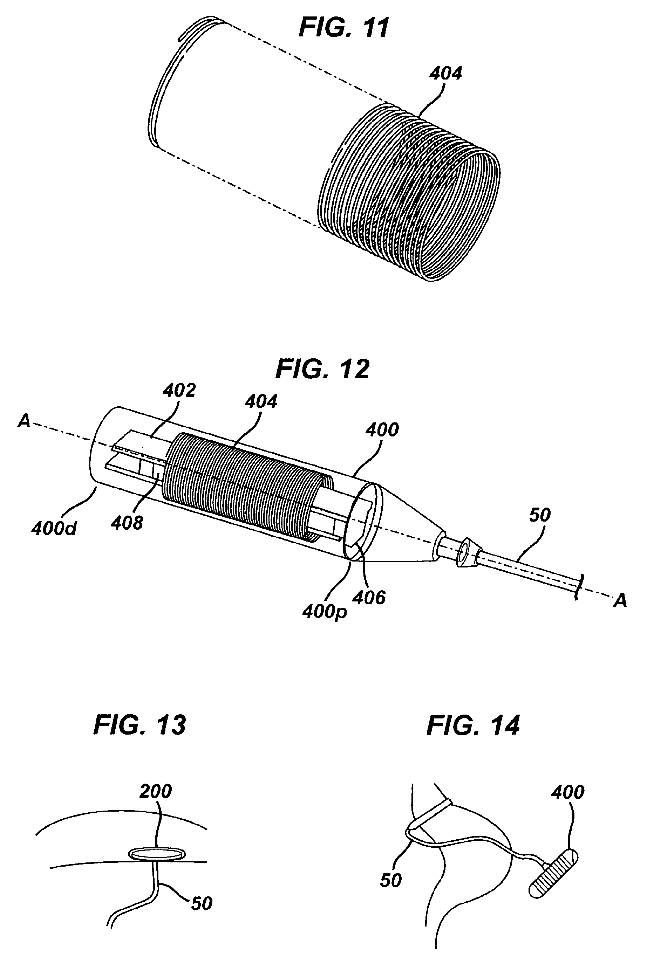

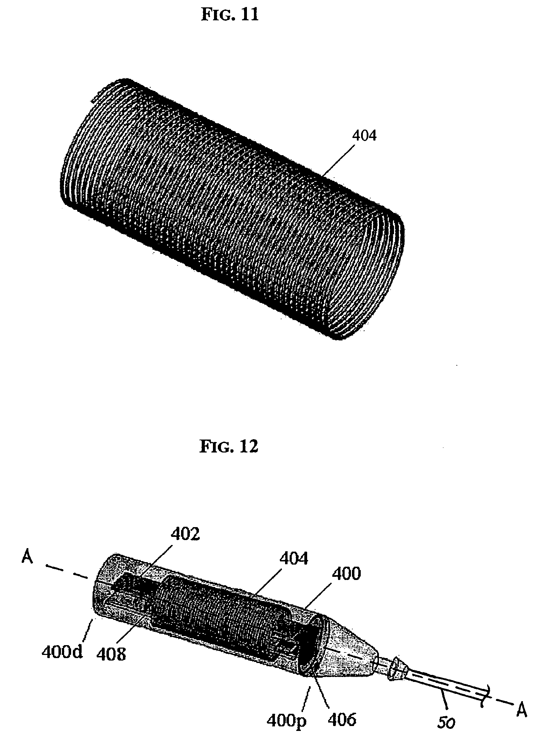

In another exemplary embodiment, as shown in FIGS. 11-12, the antenna can be in the form of a cylindrical coil antenna 404 having a longitudinal axis A that is aligned with a longitudinal axis of the catheter 50 extending from the housing 400. The cylindrical coil antenna 404 has a length and diameter that are configured to allow the antenna 404 to be disposed within the housing 400, and can have a variety of configurations. For example, the coil antenna 404 can be formed from a single continuous antenna 404 in a coiled configuration, or can be formed from a plurality of separate circular antennae positioned adjacent one another to form a coiled shape. A support 402 can be configured to support the cylindrical coil antenna 404, and in the illustrated embodiment is in the form of an elongate surface having a size that allows the support 402 to be disposed through the cylindrical coil antenna 404. The support 402 can have a length that allows the support 402 to extend through the length of the antenna 404 and to allow the support 402 to be coupled to the housing 400. The support 402 can be coupled to the housing in variety of ways. For example, in the illustrated embodiment, the support 402 to coupled to a proximal inner wall of the housing 400 using an attachment member 406. Circuitry 408 also contained within the housing 400, as described above, can also be attached to the housing 400 using the attachment member 406. A person skilled in the art will appreciate that the circuitry 408 can be attached to the housing a variety of ways, including through the use of a separate attachment member. In the illustrated embodiment, the attachment member 406 includes first and second extensions extending therefrom. The first extension is configured to couple to the support 402 to couple the support 402 to the attachment member 406, and the second extension is configured to couple to the circuitry 408 to couple the circuitry 408 to the attachment member 406. In order to facilitate communication with an external device, the field lines created by the cylindrical coil antenna 404 run substantially parallel to a longitudinal axis of the catheter 50, allowing communication with the external device regarding of the rotational orientation of the housing 400 about an axis of the catheter 50 extending therefrom.

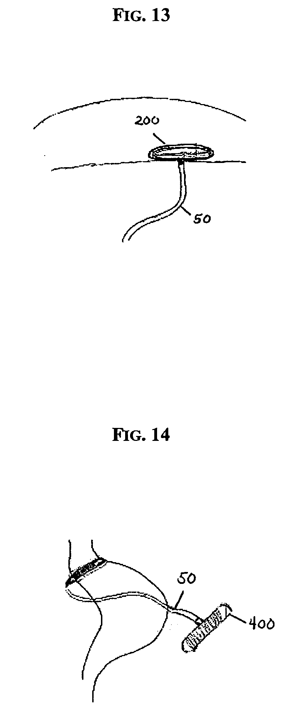

While the catheter 50 illustrated in FIGS. 8 and 12 is shown to be in line with and parallel to the antenna disposed within the housings 200, 400, FIGS. 13-14 illustrate another exemplary non-claimed embodiments of the housings 200, 400 having the antenna positioned perpendicular to the catheter 50. Moreover, while the antenna illustrated in FIGS. 8 and 12 are shown to be located within a housing also including the sensor, FIGS. 13-14 illustrate the antenna located a within a housing of an injection port 30. In order to facilitate communication with an external or internally deliverable device, the field lines created by the antenna shown in FIG. 13 or the cylindrical coil antenna shown in FIG. 14 are emitted in substantially all directions, allowing communication with the external or internal device regarding of the rotational orientation of the housing about any axis. A person skilled in art will appreciate that the antenna can be located within any housing within the restriction system to allow the antenna to communicate with an external or internally delivered device.

In use, the restriction system 10 shown in FIGS. 1A-1B can be implanted under the skin using techniques known in that art. For example, the gastric band 20 can be introduced into the patient's body and positioned around the stomach to restrict the pathway into the stomach, thus limiting food intake. The housing 60 (or 200, 400) and the port 30 can be implanted in tissue, preferably in the fascia, and they can be coupled to the band 20 to allow fluid communication therebetween. Preferably, the port 30 is anchored to a surface of the fascia, such that the port 30 is substantially parallel to the skin surface to allow access to the port 30. The housing 60, 200, 400, which is spaced a distance apart from the port 30 and preferably positioned on the fascia, can be coupled to the port 30 with the catheter 50.

After implantation, it is necessary to be able to communicate with the implantable portion 10a of the restriction system 10, for example, to transmit power to the restriction system and/or communicate system information to and from the restriction system 10. The antennae are configured within the housing, for example, the housing of the sensor or the injection port, in any of the configurations described above in order to facilitate communication with an external device. The magnetic field lines emitted and/or received by the implanted antenna are emitted and/or received in such a manner as to allow an external antenna on the external device or an internal antenna on an internally delivered device to communicate with the implanted antennae regardless of the orientation of the antennae and the housing in which they are disposed about any axis. The implantable antenna can communicate with the external antenna of the external device or the internal antenna of the internally delivered device thereby allowing the implantable system to be powered and/or various system and/or physiological parameters (e.g., pressure readings) to be transmitted and/or received from the implantable antenna to/from the external or internal antenna.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present invention.

Preferably, the invention described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The
radiation kills bacteria on the instrument and in the container. The sterilized instrument can then be stored in the sterile container. The sealed container keeps the instrument sterile until it is opened in the medical facility.

It is preferred that device is sterilized. This can be done by any number of ways known to those skilled in the art including beta or gamma radiation, ethylene oxide, steam. One of ordinary skill in the art will appreciate further features and advantages of the invention based on the above-described embodiments. Accordingly, the invention is not to be limited by what has been particularly shown and described, except as indicated by the appended claims.

## Claims

1. An implantable restriction system (10), comprising:
an implantable restriction device (20) configured to form a restriction in a pathway;
an injection port (30) configured to receive fluid therein;
a catheter (50) having a first portion and a second portion;
an implantable housing (60, 200, 400) associated with the implantable restriction device,
wherein the first portion of the catheter is coupled between the implantable restriction device and the implantable housing and the second portion of the catheter is coupled between the implantable housing and the injection port, the implantable housing having at least one antenna (114, 204, 304a, 304b, 304c, 404) configured to communicate telemetrically with a transceiver (142, 158) regardless of a rotational orientation of the housing about a longitudinal axis of the catheter, wherein the at least one antenna extends along an axis aligned with the longitudinal axis of the catheter extending from the housing.

2. A system comprising the implantable system of claim 1 and the transceiver (142), wherein the transceiver is an external device located adjacent a tissue surface.

3. A system comprising the implantable system of claim 1 and the transceiver (158), wherein the transceiver is disposed on a device configured to be delivered internally within a patient's body.

4. The system of claim 1, wherein the implantable housing includes a support (202,222,302) disposed therein having proximal and distal ends (202p; 202d) and extending along the longitudinal axis of the catheter, and wherein the at least one antenna (204, 304a, 304b, 304c) comprises a plurality of antennae with each antenna disposed around the proximal and distal ends of the support and spaced radially about the support from an adjacent antenna.

5. The system of claim 4, wherein the plurality of antennae are spaced radially apart from one another by about 180 degrees.

6. The system of claim 4, wherein the plurality of antennae are spaced radially apart from one another by about 120 degrees.

7. The system of claim 1, wherein the at least one antenna comprises a cylindrical coil antenna (404).

8. The system of claim 1, wherein the implantable housing contains a sensor (62).

9. The system of claim 8, wherein the sensor is configured to measure at least one of a system parameter and a physiological parameter, and the at least one antenna is effective to communicate the measured parameter to the transceiver.

10. The system of claim 8, wherein the at least one antenna is configured to receive energy to power the sensor (62).

11. The implantable restriction system of claim 1, wherein the implantable restriction device is an implantable band (20); and comprising
an implantable sensor (62) configured to measure at least one of a restriction system parameter and a physiological parameter; and
wherein the at least one antenna (204) associated with the housing is configured to emit a magnetic field toward an external device positioned on a tissue surface directly adjacent the housing regardless of a rotational orientation of the housing about the longitudinal axis of the catheter extending from the housing.

12. The system of claim 11, wherein the sensor (62) is configured to measure a fluid pressure of fluid in the band.

## Patentansprüche

1. Implantierbares Restriktionssystem (10), umfassend:
eine implantierbare Restriktionsvorrichtung (20), die zur Bildung einer Restriktion in einem Gang ausgelegt ist;
eine Injektionsanschlussöffnung (30), die zur Aufnahme von Flüssigkeit darin ausgelegt ist;
einen Katheter (50) mit einem ersten Abschnitt und einem zweiten Abschnitt;
ein implantierbares Gehäuse (60, 200, 400) in Zusammenhang mit der implantierbaren Restriktionsvorrichtung,
wobei der erste Abschnitt des Katheters zwischen der implantierbaren Restriktionsvorrichtung und dem implantierbaren Gehäuse angebracht ist und der zweite Abschnitt des Katheters zwischen dem implantierbaren Gehäuse und der Injektionsanschlussöffnung angebracht ist, wobei das implantierbare Gehäuse wenigstens eine Antenne (114, 204, 304a, 304b, 304c, 404) aufweist, die zur telemetrischen Kommunikation mit einem Sende-Empfangsgerät (142, 158) ausgelegt ist, unabhängig von einer Drehorientierung des Gehäuses um eine Längsachse des Katheters, wobei sich die wenigstens eine Antenne entlang einer Achse erstreckt, die mit der Längsachse des Katheters, die sich vom Gehäuse erstreckt, ausgerichtet ist.

2. System umfassend das implantierbare System nach Anspruch 1 und das Sende-Empfangsgerät (142), wobei das Sende-Empfangsgerät eine externe Vorrichtung ist, die neben einer Gewebeoberfläche angeordnet ist.

3. System umfassend das implantierbare System nach Anspruch 1 und das Sende-Empfangsgerät (158), wobei das Sende-Empfangsgerät auf einer Vorrichtung angeordnet ist, die ausgelegt ist, im Inneren des Körpers eines Patienten abgegeben zu werden.

4. System nach Anspruch 1, wobei das implantierbare Gehäuse eine Stütze (202, 222, 302) aufweist, die darin angeordnet ist und proximale und distale Enden (202p; 202d) aufweist und sich entlang der Längsachse des Katheters erstreckt, und wobei die wenigstens eine Antenne (204, 304a, 304b, 304c) eine Vielzahl von Antennen umfasst, wobei jede Antenne um die proximalen und distalen Enden der Stütze und in radialem Abstand um die Stütze von einer benachbarten Antenne angeordnet ist.

5. System nach Anspruch 4, wobei die Vielzahl von Antennen in einem radialen Abstand um ungefähr 180 Grad voneinander angeordnet sind.

6. System nach Anspruch 4, wobei die Vielzahl von Antennen in einem radialen Abstand um ungefähr 120 Grad voneinander angeordnet sind.

7. System nach Anspruch 1, wobei die wenigstens eine Antenne eine zylinderförmige Spulenantenne (404) umfasst.

8. System nach Anspruch 1, wobei das implantierbare Gehäuse einen Sensor (62) umfasst.

9. System nach Anspruch 8, wobei der Sensor zur Messung wenigstens eines Systemparameters und/oder eines physiologischen Parameters ausgelegt ist, und wobei die wenigstens eine Antenne wirksam ist, um den gemessenen Parameter an das Sende-Empfangsgerät zu kommunizieren.

10. System nach Anspruch 8, wobei die wenigstens eine Antenne ausgelegt ist, Energie zum Antreiben des Sensors (62) zu empfangen.

11. Implantierbares Restriktionssystem nach Anspruch 1, wobei die implantierbare Restriktionsvorrichtung ein implantierbares Band (20) ist; und umfassend
einen implantierbaren Sensor (62), der zur Messung wenigstens eines Restriktionssystemparameters und/oder eines physiologischen Parameters ausgelegt ist; und
wobei die wenigstens eine Antenne (204) in Zusammenhang mit dem Gehäuse ausgelegt ist, ein Magnetfeld zu einer externen Vorrichtung hin auszusenden, die auf einer Gewebeoberfläche direkt neben dem Gehäuse angeordnet ist, unabhängig von einer Drehorientierung des Gehäuses um die Längsachse des Katheters, die sich von dem Gehäuse erstreckt.

12. System nach Anspruch 11, wobei der Sensor (62) zur Messung eines Flüssigkeitsdrucks von Flüssigkeit im Band ausgelegt ist.

## Revendications

1. Système de restriction implantable (10), comprenant :
un dispositif de restriction implantable (20) configuré pour former une restriction dans une voie ;
un port d'injection (30) configuré pour y recevoir un fluide ;
un cathéter (50) ayant une première partie et une seconde partie ;
un boîtier implantable (60, 200, 400) associé au dispositif de restriction implantable, où la première partie du cathéter est couplée entre le dispositif de restriction implantable et le boîtier implantable et la seconde partie du cathéter est couplée entre le boîtier implantable et le port d'injection, le boîtier implantable comportant au moins une antenne (114, 204, 304a, 304b, 304c, 404) configurée pour communiquer par télémétrie avec un émetteur-récepteur (142, 158) indépendamment d'une orientation rotationnelle du boîtier autour d'un axe longitudinal du cathéter, où l'au moins une antenne s'étend le long d'un axe aligné avec l'axe longitudinal du cathéter s'étendant le long du boîtier.

2. Système comprenant le système implantable de la revendication 1 et l'émetteur-récepteur (142), où l'émetteur-récepteur est un dispositif externe situé de manière adjacente par rapport à une surface de tissu.

3. Système comprenant le système implantable de la revendication 1 et l'émetteur-récepteur (158), où l'émetteur-récepteur est disposé sur un dispositif configuré pour être délivré en interne à l'intérieur du corps d'un patient.

4. Système selon la revendication 1, dans lequel le boîtier implantable comprend un support (202, 222, 302) y étant disposé, comprenant des extrémités proximale et distale (202p ; 202d) et s'étendant le long de l'axe longitudinal du cathéter, et où l'au moins une antenne (204, 304a, 304b, 304c) comprend une pluralité d'antennes, avec chaque antenne disposée autour des extrémités proximale et distale du support et espacée radialement d'une antenne adjacente autour du support.

5. Système selon la revendication 4, dans lequel la pluralité d'antennes sont espacées radialement les unes des autres d'environ 180 degrés.

6. Système selon la revendication 4, dans lequel la pluralité d'antennes sont espacées radialement les unes des autres d'environ 120 degrés.

7. Système selon la revendication 1, dans lequel l'au moins une antenne comprend une antenne à cadre cylindrique (404).

8. Système selon la revendication 1, dans lequel le boîtier implantable contient un capteur (62).

9. Système selon la revendication 8, dans lequel le capteur est configuré pour mesurer au moins un paramètre parmi un paramètre de système et un paramètre physiologique, et l'au moins une antenne est efficace pour communiquer le paramètre mesuré à l'émetteur-récepteur.

10. Système selon la revendication 8, dans lequel l'au moins une antenne est configurée pour recevoir de l'énergie pour alimenter le capteur (62).

11. Système de restriction implantable selon la revendication 1, dans lequel le dispositif de restriction implantable est une bande implantable (20) ; et comprenant :
un capteur implantable (62) configuré pour mesurer au moins un paramètre parmi un paramètre de système de restriction et un paramètre physiologique ; et
où l'au moins une antenne (204) associée au boîtier est configurée pour émettre un champ magnétique vers un dispositif externe positionné sur la surface d'un tissu de manière directement adjacente au boîtier indépendamment d'une orientation rotationnelle du boîtier autour de l'axe longitudinal du cathéter s'étendant depuis le boîtier.

12. Système selon la revendication 11, dans lequel le capteur (62) est configuré pour mesurer une pression de fluide du fluide dans la bande.
